# EUROPEAN PATENT APPLICATION

(11) **EP 2 305 700 A1**
(43) Date of publication of application: **06.04.2011**
(21) Application number: 10177682.1
(22) Date of filing: 01.10.2003
(51) Int. Cl.: C07K 14/195, G01N 33/00, A61P 11/00, A61P 35/00, A61P 31/04

(54) **Therapeutic USPA1-derived peptides**

(30) Priority: 02.10.2002 GB 0222764
(62) Divisional of application: 03756554.6
(71) Applicant: UNIVERSITY OF BRISTOL, Bristol BS8 1TR (GB)
(72) Inventor: Virji, Mumtaz, Bristol, BS8 1TD (GB)
(74) Representative: McQueen, Andrew Peter

(57) **Abstract**

The present invention provides a ligand isolated from *Moraxella catarrhalis* outer membrane protein which binds to CEACAM receptors, said ligand comprising a receptor binding domain comprising an amino acid sequence selected from the group disclosed, or a fragment, homologue, functional equivalent, derivative, degenerate or hydroxylation, sulphonation or glycosylation product or other secondary processing product thereof.

The invention also provides medicaments and vaccines comprising said ligand, and their use in the treatment or prophylaxis of infection. Also provided is a screening method for the identification of novel therapeutic compounds.

## Description

This invention relates to therapeutic peptides and in particular to therapeutic peptides which are useful in the preparation of vaccines or other treatments for infection as well as in the screening of compounds for potential pharmaceutical activity in the treatment of infections.

The present inventors have previously identified that Carcinoembryonic antigen related cell adhesion molecules (CEACAMs) are receptors for pathogens of mucosal membranes, especially for respiratory pathogens such as *Neisseria meningitidis, Haemophilus influenzae* and *Moraxella catarrhalis.* CEACAMs belong to the CarcinoEmbryonic Antigen (CEA) family, a member of the Immunoglobulin superfamily. The CEA gene family comprises surface expressed (CEA) and secreted (pregnancy-specific glycoprotein, PSG) subfamilies. The membrane-associated sub-family redefined as CEACAM (CEA-related cell adhesion molecule) ²⁰ comprises several related glycoproteins of which CEACAM1 is the most widely expressed in distinct human tissues ¹². The studies reported by the inventors primarily used Chinese Hamster Ovary (CHO) cells transfected with CEACAM1 (previously termed CD66a and BGPc) that contains four extracellular domains, a TM region and a short (S) or a long (L) cytoplasmic tail (molecular formula: NA1BA2-TM-S or L). In addition, soluble truncated constructs containing one or more of the extracellular domains were used. Previous studies demonstrated that both *Neisseria meningitidis* and *Haemophilus influenzae* primarily target the N-domain of several CEACAMs ^{7,9,10}. Such targeting may lead to cell surface attachment as well as cellular invasion ⁹. In addition, bacteria may bind to CEACAMs on phagocytic cells and T and B lymphocytes. Such interactions may lead to bacterial cell death ⁸, target cell death or inhibition of immune function, e.g. of T and B lymphcytes when *N. gonorrhoeae* (closely related to *N. meningitidis)* binds to CEACAMs of these lymphocytes ^{21,22}_{.}

The presence of CEACAM-binding ligands in *Moraxella catarrhalis* and *Haemophilus influenzae* has been a surprising find to the present inventors since CEACAMs have long been associated with outer membrane opacity-associated Opa proteins of *Neisseriae* and neither *Haemophilus influenzae* nor *Moraxella catarrhalis* produce Opa proteins. In the description which follows, the present invention will be described with particular reference to infection of the mucosal membranes, especially of the respiratory membranes, or to infection of the ear (especially otitis media) but it is to be understood that the invention finds equal utility in other areas such as the genital mucosa or the urethrae where CEACAM receptors are implicated in infection or other receptor-binding processes or elsewhere in human infection where bacteria may become disseminated from mucosal surfaces.

The mucosal pathogens *Neisseria meningitidis* (Nm), *Haemophilus influenzae* (Hi) and *Moraxella catarrhalis* (Mx) are human specific organisms and reside in the upper respiratory tract from where they may disseminate to cause serious infections. Meningococcal strains of distinct serogroups may be carried within the nasopharynx of up to 25% of healthy individuals¹. However, in a number of subjects, the organism invades the mucosal barrier to cause one of the most rapidly advancing and extremely serious diseases. The precise factors that increase host susceptibility to meningococcal infection are not fully understood. Moreover, the limited protection afforded by group-specific vaccines and the non-immunogenicity of the group B polysaccharide underscore the need for fundamental studies to understand host susceptibility and identify salient sub-capsular features that could serve as common targets to combat meningococci. Studies by the present inventors have provided an understanding of the molecular basis of meningococcal colonisation, the nature of its interactions with human barrier cells (epithelial and endothelial) as well as phagocytic cells. In recent years, the basis for mucosal colonisation by commensal *Neisseriae* has been investigated to understand the features which differentiate between largely harmless colonisers and occasional but serious pathogens such as Nm. In addition, the studies have determined whether commensal *Neisseriae* could be used as carriers of potential vaccine antigens of Nm.

Up to 75% of healthy individuals may carry strains belonging to the species *H. influenzae*². Although as a result of the Hib vaccine, there has been a dramatic decrease in the incidence of the type b disease in the West, diseases caused by non-typable Hi (NTHi) strains remain a major problem. NTHi cause localised as well as disseminated infections including epiglottitis, otitis media, cellulitis, pneumonia, endocarditis, bacteraemia and meningitis. Otitis media is one of the major problems in paediatric medicine and NTHi are responsible for over 20% of episodes in children during the first year of life ^{2,3}. NTHi are also associated with acute recurrent and persistent infections in patients with chronic obstructive pulmonary disease (COPD) and cystic fibrosis. What determines recurrent infections by NTHi in these patients or multiple episodes of otitis media in children is unclear ^{2,3,4}.

*Moraxella catarrhalis,* another resident of the human respiratory tract, is often isolated from cases of localised infections together with Hi. Both organisms are associated with sinusitis and exacerbations of asthmatic conditions ^{5,6}. Mx is the third most common cause of otitis media in children (estimated to be responsible for 3-4 million cases annually). It also causes lower respiratory tract infections in adults especially in patients with COPD ⁵. On rare occasions, it has been associated with disseminated infections ⁵. Both Hi and Mx cause persistent infections and are believed to escape host immune mechanisms and antibiotics by tissue penetration ⁴. Several outer membrane proteins of Mx have been studied with respect to their adhesive properties. However, few cellular receptors for Mx have been identified and many of the details of pathogenic mechanisms remain to be investigated ^{4,5,6}.

A primary requirement for respiratory mucosal pathogens is establishment of firm contact with respiratory epithelial cells. The targets of these human tropic pathogens are human specific molecules and studies have to rely on *in vitro* human tissue and organ cultures. The attachment is often mediated by bacterial phase- and antigenically-variable structures. In addition, it is becoming increasingly clear that attachment of pathogens is multi-faceted and environmental adaptation plays a significant role in the manner of attachment. Although many recent studies have begun to define various stages in the complex cellular targeting mechanisms, the details of environmental adaptation or host-microbial cross-talk remain to be described.

Recent studies by the present inventors have shown that Nm⁷⁻⁹ and Hi^{10,11} share some distinct and other common mechanisms of targeting certain human cell surface receptors CEACAMs ¹².

Furthermore, the present inventors have recently identified that clinical isolates of *Moraxella catarrhalis* also target the human CEACAM molecules. In addition, it has now been found that a Moraxella outer membrane protein of high molecular weight binds to the receptor. Expression of CEACAMs in distinct tissues¹² including respiratory epithelial cells has been demonstrated ²⁴. These observations imply that specific targeting of CEACAMs is of particular advantage to respiratory bacteria and may have arisen as a result of convergent evolution.

Amongst the adhesion factors elaborated by *Neisseria meningitidis* are pili (fimbriae)^{13,14,16} and the outer membrane opacity proteins, Opa and Opc ^{15,16}. Nm pili are long filamentous protein structures composed of multiple pilin subunits. They are generally regarded as the most important adhesins in capsulate bacteria ^{13,14,16} due the fact that capsule partly or totally masks outer membrane ligands resulting in their reduced functional efficacy, whilst pili traverse the capsule and remain functional in fully capsulate bacteria. Opa are antigenically variable family of proteins and occur *in N. meningitidis* as well as *N. gonorrhoeae.* In meningococci, 3 - 4 *opa* gene loci code for related transmembrane proteins with four surface exposed loops, three of which undergo sequence variation ^{16,17}. Opc, another trans-membrane protein, is largely invariant ^{15,16} Over the last 12 years, the present inventors have investigated structure/function relationships of Nm pili, the virulence potential of Opa and Opc proteins and identified two human receptors for the neisserial opacity proteins. Further, the role of surface sialic acids in bacterial interactions with human target cells as well as the role of LPS and other factors in cellular toxicity have been studied by the present inventors.

The present invention results from the identification by the inventors of a ligand of high molecular weight isolated from Moraxella outer membrane protein which binds to CEACAM receptors.

The ligand can be characterised by its SDS-PAGE migration pattern which is indicative of the USP-family of proteins in that it is broken down to monomers having a molecular weight of between approximately 60 and 150 kD when boiled for a prolonged period.

The ligand has been characterised in Mx strain ATCC 25238 (MX2) as UspA1 and its amino acid sequence determined. The ligand has been further characterised to determine the receptor binding region or domain, i.e. peptide or peptide-associated features that bind to the receptor.

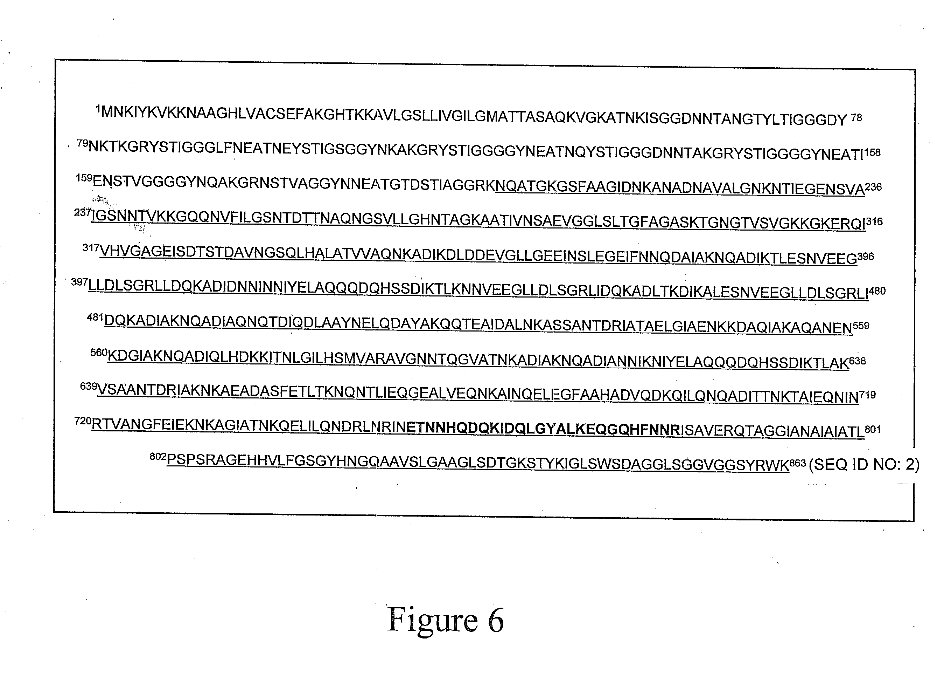

Accordingly, the present invention provides a ligand isolated from *Moraxella catarrhalis* outer membrane protein which binds to CEACAM receptors, said ligand comprising a receptor binding domain comprising an amino acid sequence selected from the group consisting of residues 463 to 863, 527 to 668, 527 to 863, 427 to 623, 427 to 668, and 427 to 863 of the sequence shown in Figure 6, or a fragment, homologue, functional equivalent, derivative, degenerate or hydroxylation, sulphonation or glycosylation product or other secondary processing product thereof.

The term ligand is used herein to denote both the whole molecule which binds to the receptor and any part thereof which includes the receptor binding domain such that it retains the receptor binding property. Thus "ligand" encompasses molecules which consist only of the receptor binding domain i.e. the peptide region or regions required for receptor binding.

In one embodiment, the present invention provides a ligand consisting of an amino acid sequence selected from the group consisting of residues 463 to 863, 527 to 668, 527 to 863, 427 to 623, 427 to 668, and 427 to 863 of the sequence shown in Figure 6, or a fragment, homologue, functional equivalent, derivative, degenerate or hydroxylation, sulphonation or glycosylation product or other secondary processing product thereof.

Structurally and/or functionally equivalent receptor binding domains may also occur in other UspA-like proteins since hybrid proteins occur in Mx that may contain mosaic epitopes derived from both UspA1 and UspA2 proteins ²³. Ligands comprising such equivalent receptor binding domains are also within the scope of the invention.

Preferably the ligand or receptor binding domain is suitable for use in the prevention or treatment of infection.

The present invention also provides a nucleic acid sequence encoding the ligand protein of the present invention together with homologues, polymorphisms, degenerates and splice variants thereof.

The ligand of the invention, or combinations thereof may be used in a vaccine or other prophylactic treatment of infection.

The vaccine or other prophylactic treatment may comprise any known adjuvant, vehicle, excipient, binder, carrier, preservatives and the like, to provide a pharmaceutically acceptable preparation of the ligand for use in the treatment of a patient.

The invention also provides a pharmaceutically acceptable preparation of the ligand for use in medicine.

The pharmaceutically acceptable preparation of the ligand may be used in the treatment or prevention of any disease where CEACAM receptors are implicated, for example in the treatment or prevention of infection, respiratory disease, neoplastic diseases and associated conditions of neoplastic diseases, and angiogenesis.

Preferably, where an infection is treated, the infection is of, or has occurred via, the mucosal membrane, especially a respiratory infection.

Most preferably, the ligand is used as a vaccine for or in other prophylaxis or treatment of *Neisseria meningitidis, Haemophilus influenzae* and *Moraxella catarrhalis.* Ideally, the ligand is used as a vaccine for or in other prophylaxis or treatment of otitis media.

In a further aspect, the ligand of the present invention may also be used to identify novel blocking reagents for use as therapeutic agents to protect vulnerable groups and the public in general against several mucosal pathogens. For example the ligand may be used to identify receptor analogs which are useful for this purpose.

Hence the present invention also provides a screening assay for the identification of novel blocking reagents for use as therapeutic agents, the assay comprising the steps of screening potential therapeutic agents for their ability to mimic or for their homology to the ligand of the present invention. The invention further provides therapeutic agents identified by the aforementioned screening assay.

Effective vaccine components may be produced by using the information of the receptor targeting mechanisms identified by the present invention such as biologically active peptide mimics. These could prevent bacterial colonisation / invasion of mucosa as well as elicit antibodies which may be blocking, opsonic and bactericidal.

Bacterially derived biologically active peptide sequences identified by the ligand of the present invention could be used to study the roles of CEACAMs in cancer and development as the molecules are associated with these processes. These also have potential as anti-cancer agents and to control or otherwise in the treatment of angiogenesis.

In a further embodiment, the invention provides the use of a CEACAM receptor-binding ligand in the manufacture of a medicament for the treatment or prophylaxis of a disease in which CEACAM receptors are involved in cellular targeting of the pathogen which causes the disease, wherein the ligand comprises an amino acid sequence selected from the group consisting of residues 463 to 863, 527 to 668, 527 to 863, 427 to 623, 427 to 668, and 427 to 863 of the sequence shown in Figure 6, or a fragment, homologue, functional equivalent, derivative, degenerate or hydroxylation, sulphonation or glycosylation product or other secondary processing product thereof.

Preferably, the disease is selected from the group consisting of infection, respiratory disease, neoplastic disease and associated conditions of neoplastic disease, and angiogenesis.

Medicaments as described above are of particular utility where the pathogen infects, or enters via, a mucosal membrane.

Medicaments as described herein are particularly useful in the treatment or prevention of infections (disease) caused by *Moraxella catarrhalis.* However, ligands as described herein are useful in the manufacture of medicaments for the treatment of any disease where CEACAM receptors are implicated such as diseases caused by *Neisseria meningitidis* and *Haemophilus influenzae.*

In a particularly preferred embodiment, the disease is otitis media.

Ligands of the invention may also be used in the treatment of diseases caused by other oral bacteria, such as dental caries.

Accordingly, the present invention is directed to the following embodiments:
1. A ligand comprising a CEACAM receptor binding domain, said ligand consisting of an amino acid sequence selected from the group consisting of residues 463 to 863, 527 to 668, 527 to 863, 427 to 623, 427 to 668, and 427 to 863 of the sequence shown in Figure 6, or a fragment, homologue, functional equivalent, derivative, degenerate or hydroxylation, sulphonation or glycosylation product or other secondary processing product thereof.
2. A medicament comprising the ligand according to paragraph 1 and one or more pharmaceutically acceptable adjuvants, vehicles, excipients, binders, carriers, or preservatives.
3. Use of the medicament according to paragraph 2 for the treatment or prophylaxis of infection.
4. Use of the medicament according to paragraph 2 for the treatment or prevention of any disease where CEACAM receptors are implicated.
5. Use according to paragraph 4 for the treatment or prevention of infection, respiratory disease, neoplastic diseases and associated conditions of neoplastic diseases, and angiogenesis.
6. Use according to any one of paragraphs 3 to 5, where the infection is of, or has occurred via, a mucosal membrane.
7. Use according to paragraph 6, wherein the infection is caused by *Neisseria meningitidis, Haemophilus influenzae* or *Moraxella catarrhalis.*
8. Use of the medicament according to paragraph 2 for the prophylaxis or treatment of otitis media.
9. A screening assay for the identification of novel blocking reagents for use as therapeutic agents comprising screening potential therapeutic agents for their ability to mimic, or for their homology to, the ligand according to paragraph 1.
10. A vaccine comprising the ligand according to paragraph 1 and one or more pharmaceutically acceptable adjuvants, vehicles, excipients, binders, carriers, or preservatives.
11. A method of treating or preventing infection in an individual in need thereof comprising administering an effective amount of a medicament according to paragraph 2.
13. A ligand substantially as hereinbefore described with reference to and as illustrated by Figure 6 of the accompanying drawings.
14. A medicament substantially as hereinbefore described comprising the ligand of paragraph 13.
15. Use of a CEACAM receptor-binding ligand in the manufacture of a medicament for the treatment or prophylaxis of a disease in which CEACAM receptors are involved in cellular targeting of the pathogen which causes the disease, wherein the ligand comprises an amino acid sequence selected from the group consisting of residues 463 to 863, 527 to 668, 527 to 863, 427 to 623, 427 to 668, and 427 to 863 of the sequence shown in Figure 6, or a fragment, homologue, functional equivalent, derivative, degenerate or hydroxylation, sulphonation or glycosylation product or other secondary processing product thereof.
16. Use according to paragraph 15 wherein the disease is selected from the group consisting of infection, respiratory disease, neoplastic disease and associated conditions of neoplastic disease, and angiogenesis.
17. Use according to paragraph 15 or 16, wherein the pathogen infects, or enters via, a mucosal membrane.
18. Use according to any of paragraphs 15 to 17, wherein the pathogen which causes the disease is selected from the group consisting of *Neisseria meningitidis, Haemophilus influenzae* and *Moraxella catarrhalis.*
19. Use according to paragraph 18, wherein the disease is otitis media.

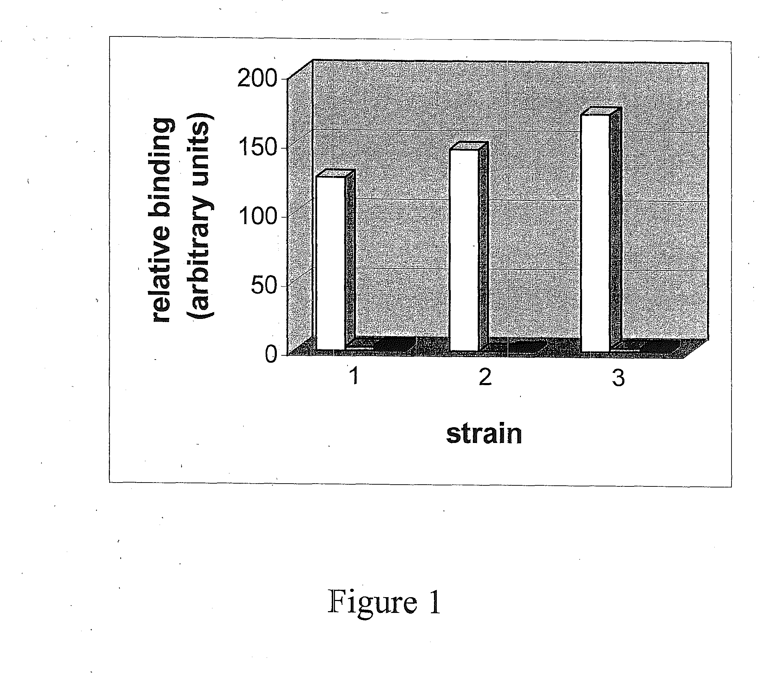

Embodiments of the present invention will now be described purely by way of non-limiting example in which reference is made to the figures of which:-
Figure 1 is a graph showing relative binding levels of CEACAM1-Fc (1µg.ml⁻¹) soluble receptor construct alone (white, left hand bars), or in the presence of the CEACAM1 N-domain specific antibody YTH71.3 (grey, centre bars) to 3 strains of Mx immobilised on nitrocellulose. CD33-Fc binding in each case was negligible (black, right hand bars). Strains 1, 2, 3: MX2 (ATCC 25238), MX3, MX4 (clinical isolates) respectively. Binding was determined in a dot blot overlay and the intensity of reactions quantified by densitometric analysis using NIH Scion Image program.

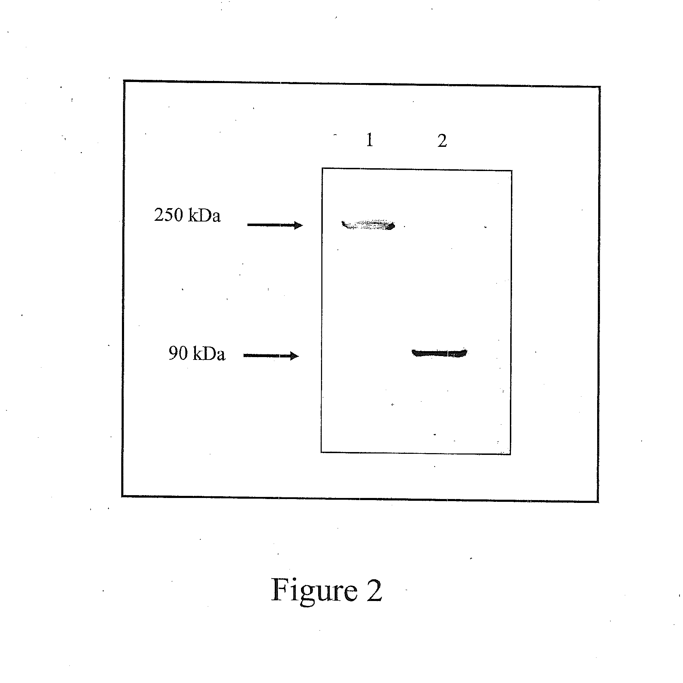

Figure 2 shows a Western blot of the strain MX2 proteins separated under undissociating (unheated, lane 1) or after boiling for 10 min. (lane 2). Blot was overlaid with CEACAM1-Fc (1 µg.ml⁻¹) and the receptor binding detected with anti-human Fc antibody conjugated to horseradish peroxidase and its substrate.

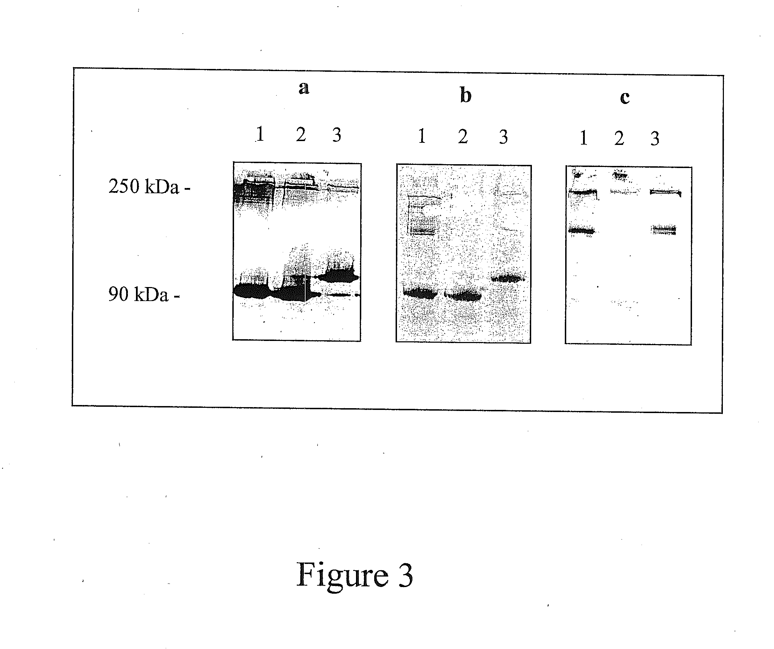

Figure 3 shows Western blots of denatured whole cell lysates of strains MX2, -3, -4 (lanes 1-3 respectively) were overlaid with CEACAM1-Fc (1 µg. ml⁻¹; a), anti-UspA1 peptide antibody (10 µg.ml⁻¹; b) and anti-UspA2 peptide antibody (10 µg.ml⁻¹; c). Note the similar migration profile of CEACAM1-Fc binding proteins and anti-UspA1 binding proteins in the three strains. Remnants of undissociated proteins at c. 250 kDa, (detected in this case due to higher sensitivity of detection in the alkaline phosphatase assay), bind to CEACAM1-Fc. These are only weakly recognized by the anti-peptide antibodies, presumably since the epitopes contained within synthetic peptides are not fully exposed in the native protein, which become progressively exposed as the complex denatures. Anti-UspA2 antibodies bind to proteins of apparent masses > 200 kDa, which remain undissociated after boiling, a property indicative of UspA2 proteins.

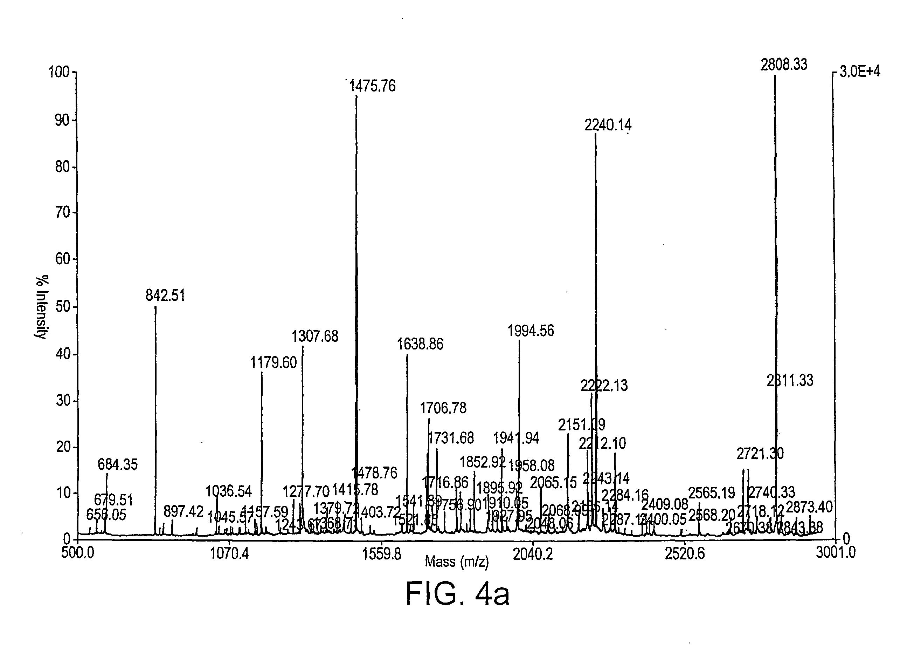

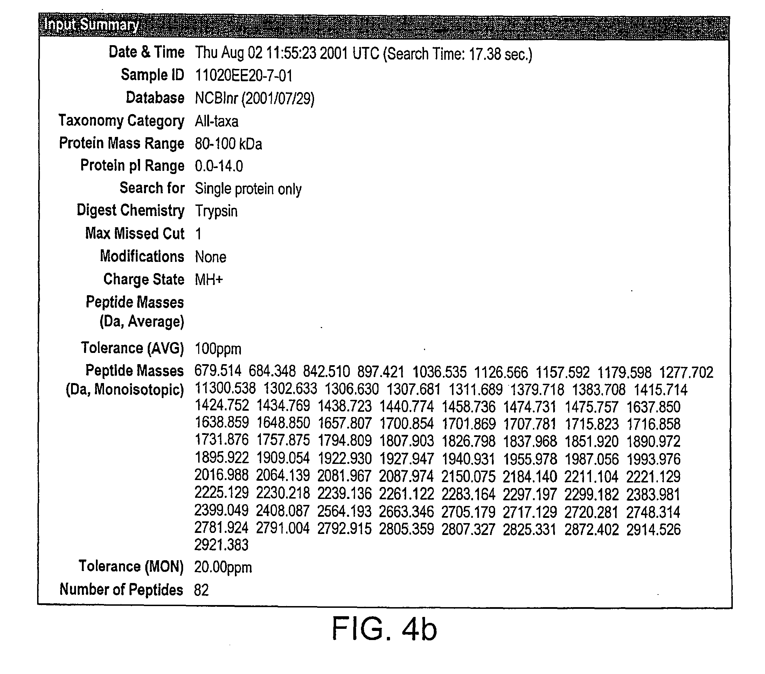

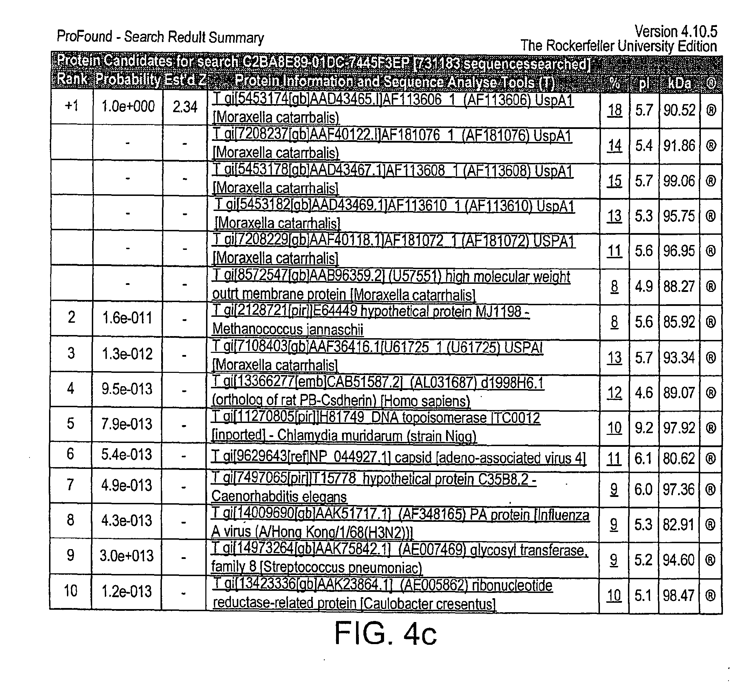

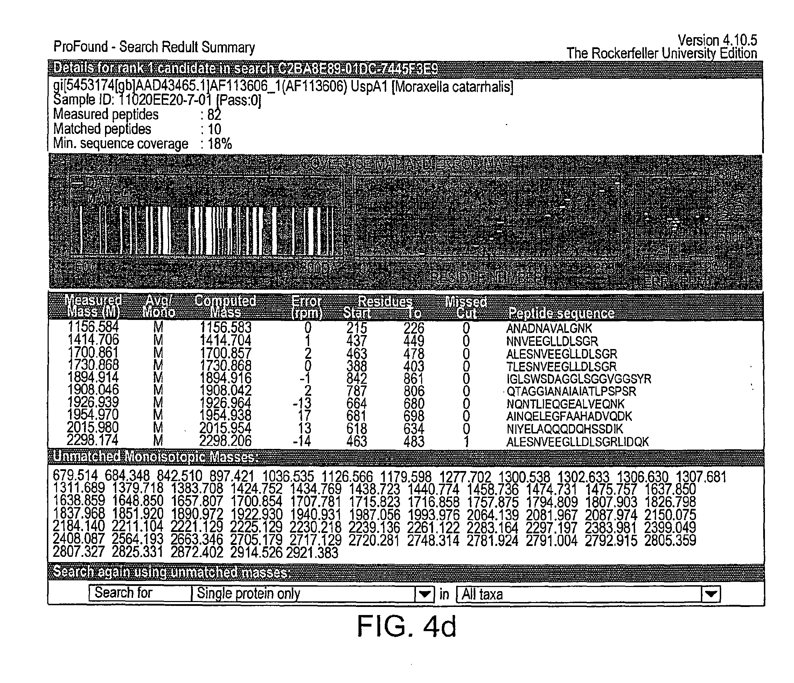

Figure 4 shows a mass spectrum of tryptic peptides of the CEACAM1 binding protein of MX2 following electro-elution (4a). The summary of data input into the ProFound protein identification database is shown in (4b). A table of the top ten identified proteins and the probability values are shown in (4c). Detail of the number 1 ranked candidate, in this case UspA1 with a Z-score of 2.34 is shown in (4d) indicating the number of peptides matched, their positions within the protein, the % of the protein covered and a list of peptides unmatched on this occasion.

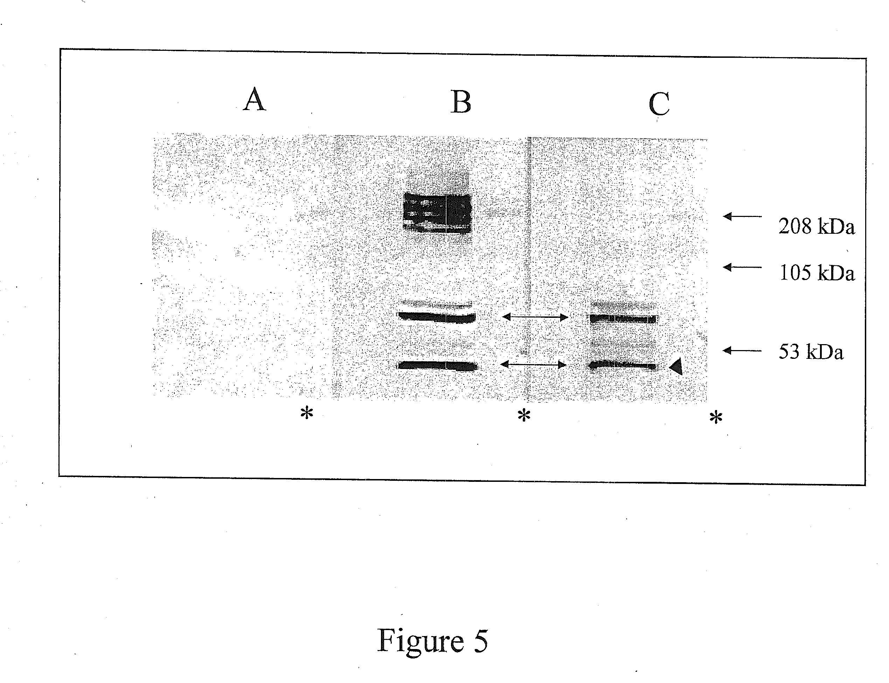

Figure 5 shows Western blot analysis of tryptic fragments of UspA1 of MX2. A: control blot using secondary antibodies (mixture of goat anti-human Fc and goat anti-rabbit Ig used in B and C). B: blot overlaid with CEACAM1-Fc and goat anti-human Fc. C: blot overlaid with affinity purified rabbit antibodies raised against UspA1 peptide (ETNNHQDQKIDQLGYALKEQGQHFNNR (SEQ ID NO:1)) (see Figure 6) and anti-rabbit Ig. * = lanes with molecular weight markers - shown on the left. The peptides shown by the double arrows react strongly with CEACAM1-Fc (B) as well as the anti-UspA1 peptide antibodies (C). The binding of the anti-UspA1 peptide antibodies to the lowest MW peptide (arrowhead) identifies this CEACAM-binding fragment as the C-terminal fragment contained within N-199 to K-863 of UspA1 of MX2 (see Figure 6).
Figure 6 shows the amino acid sequence of MX2 UspA1 protein. UspA1-specific peptide used to raise antisera in rabbits is shown in bold. The CEACAM-binding region is contained in the underlined C-terminal fragment of UspA1 of MX2.

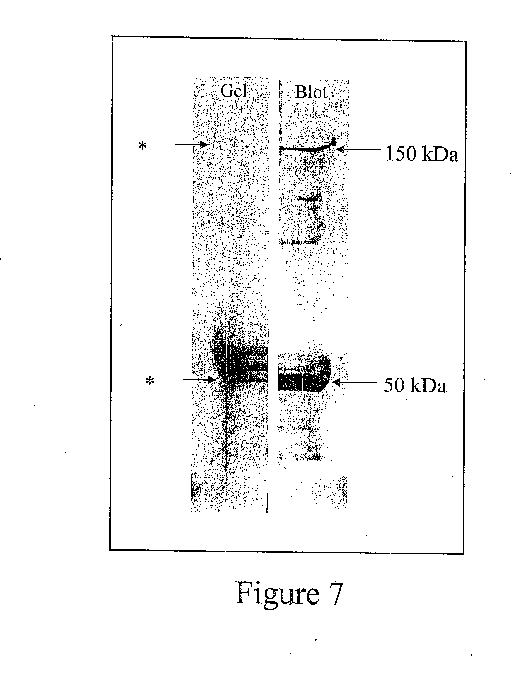

Figure 7 shows the separation of tryptic peptides reacting with CEACAMs. *M. catarrhalis* strain MX2 was treated with 1 mg/ml trypsin at 37°C for 10 min. Trypsinised sample was subjected to SDS-PAGE. After staining, 50 kDa region was electroeluted overnight. Electroeluted protein was freeze dried and resuspended in buffer and applied to a second gel. Part of the gel was blotted onto nitrocellulose and peptide bands reacting with CEACAM were identified by Western blot overlay using CEACAM1-Fc (Blot).
   '*': denotes peptide bands sent for N-terminal sequencing.

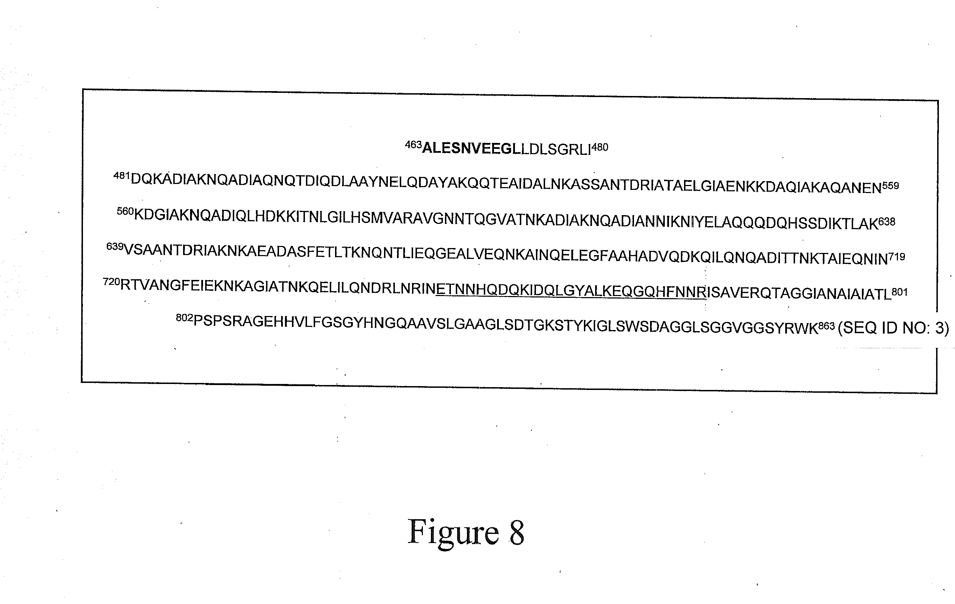

Figure 8 shows the amino acid sequence of a tryptic peptide of MX2 UspA1 protein. The 50 kDa tryptic peptide shown (amino acids 463 to 863) binds CEACAM and antiserum against UspA1 peptide (amino acids 753-780 underlined). The N-terminal sequence of the *c.* 50 kDa CEACAM binding peptide is "ALESNVEEGL" (SEQ ID NO:4) that occurs after the trypsin cleavage site at amino acid 462.

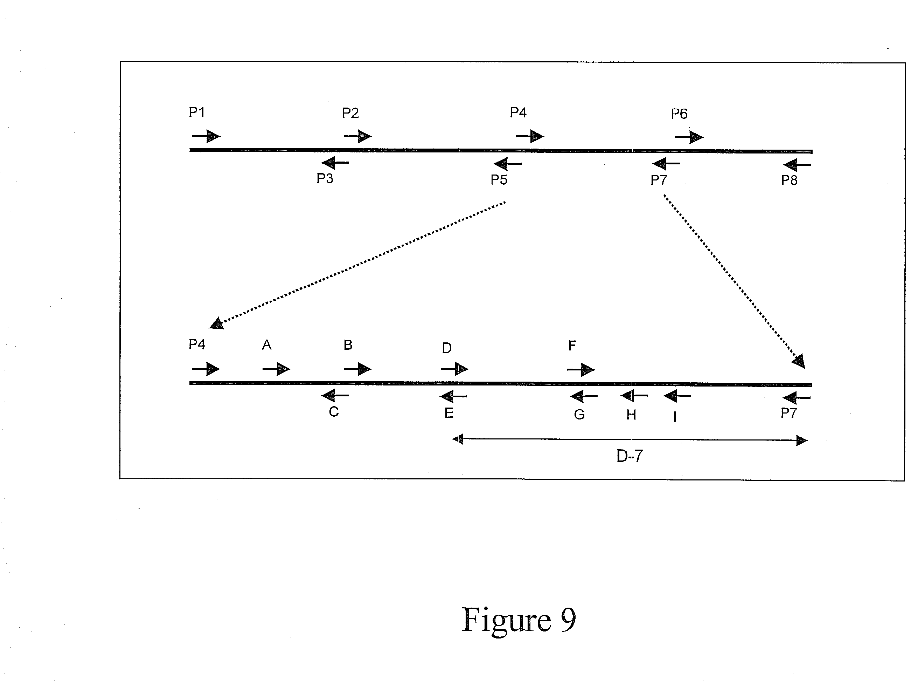

Figure 9 shows a diagrammatic representation of the positions of primers used to generate *uspA1* gene fragments for the expression of recombinant peptides. The CEACAM1 binding site was encoded by DNA amplified by primers P4 and P7, additional primers throughout this region (letters A-I) were designed and employed.

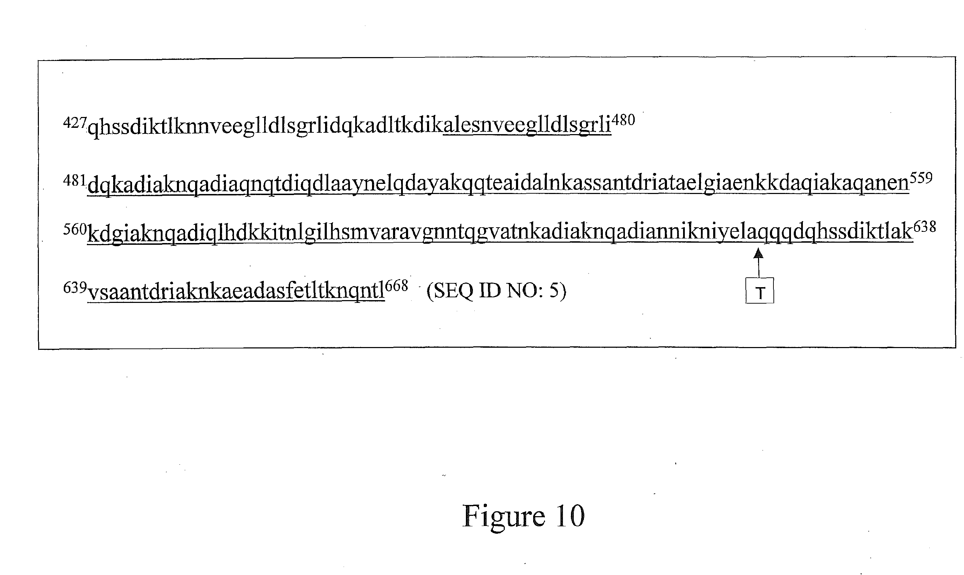

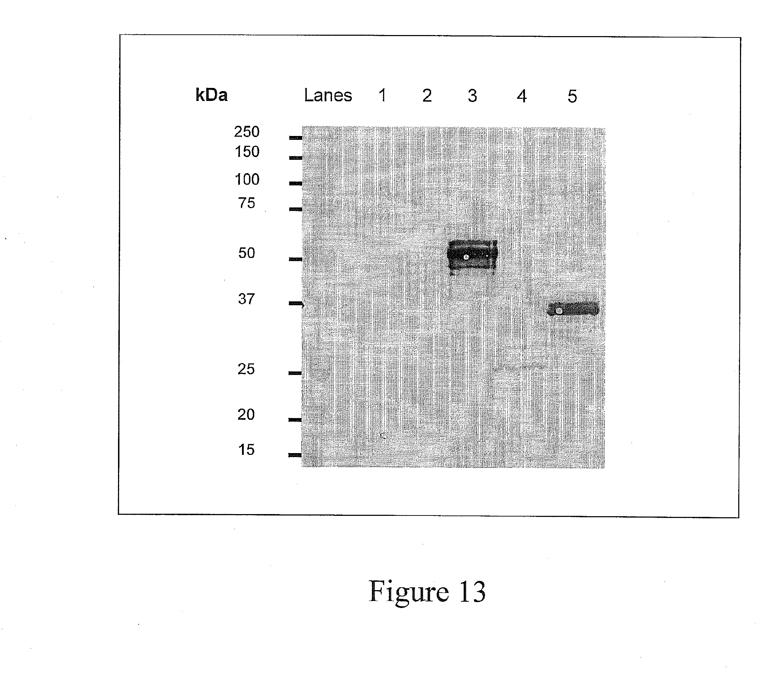

Figure 10 shows the sequence of the recombinant fragment 4-7. The underlined region is the N-terminal region of the CEACAM1-reactive tryptic peptide. The predicted molecular weight of the fragment 4-7 is c. 26 kDa. The predicted MW of the His-tagged fragment: c. 28kDa. The position of the truncated peptide is shown by 'T' (see Figure 13).

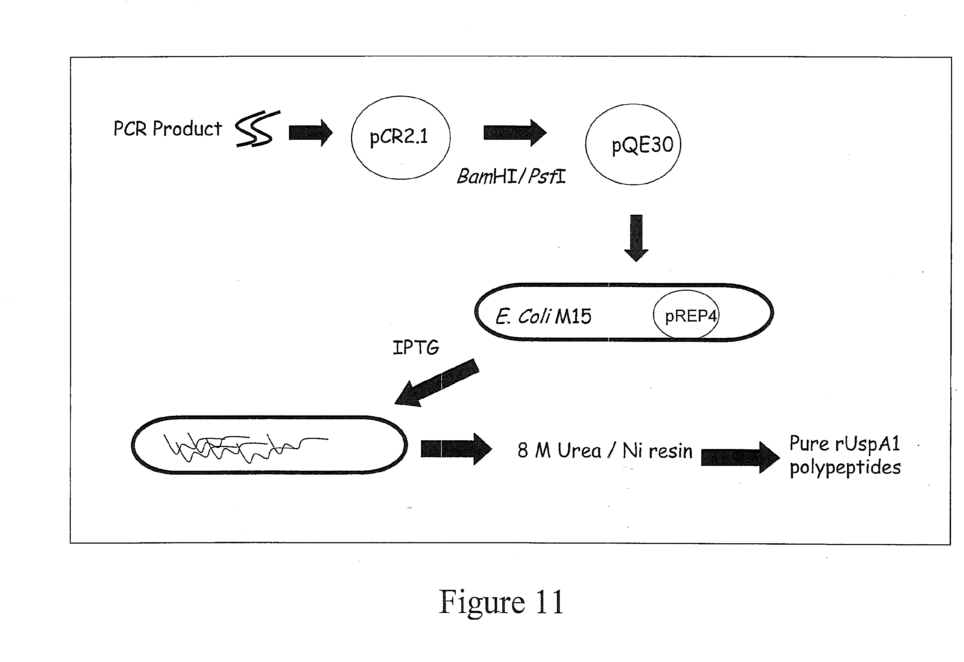

Figure 11 is a diagram showing the general cloning, expression and purification strategy for recombinant UspA1 peptides, as exemplified by the pQE30 system.

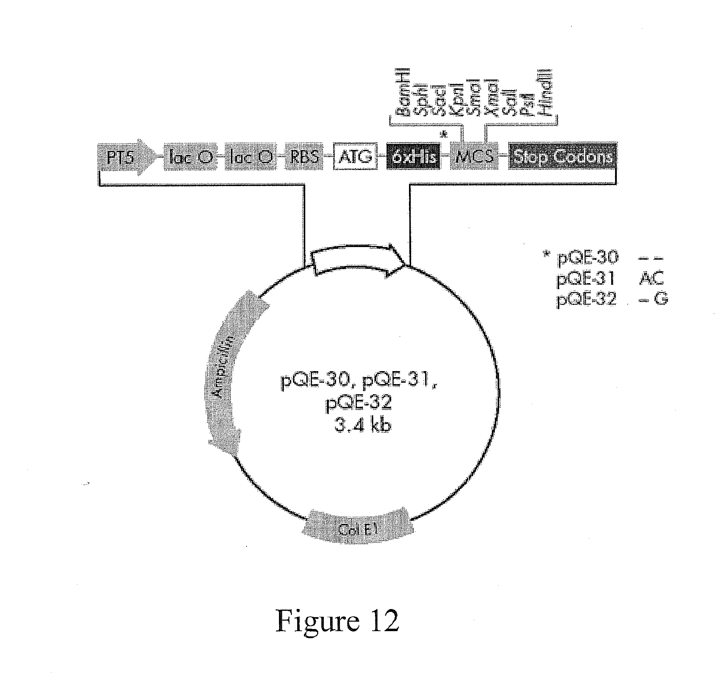

Figure 12 is a map of the vector pQE30.
Figure 13 shows binding of CEACAM1-Fc in blot overlay to recombinants 4-8 (lane 3), 4-T (lane 4) and 4-7 (lane 5) polypeptides. Lane 1 contained a Treponemal control recombinant peptide and lane 2 contained lysates of non-induced M15 containing 4-8 construct.

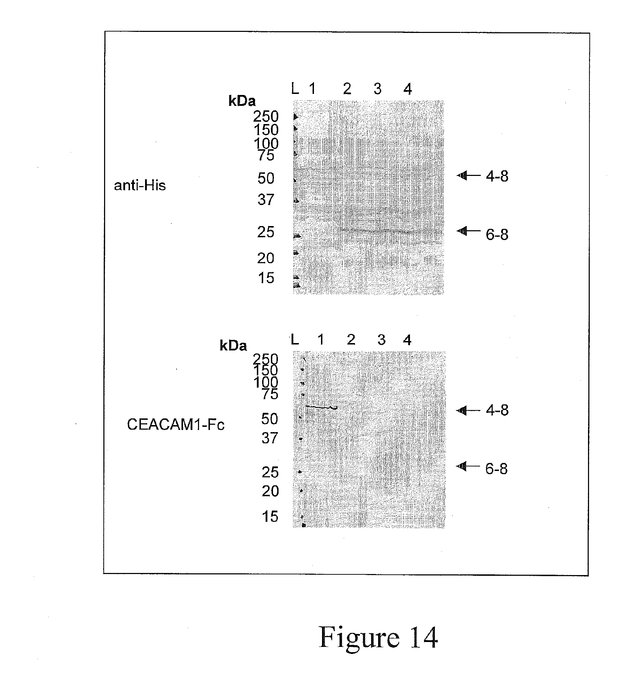

Figure 14 shows Western blots showing recombinant peptide reactivity with anti-His tag antibody (top) and CEACAM1-Fc (bottom). Lanes 2-4 contained 6-8 peptide, lane 1 contained 4-8 as a control. Predicted migration positions of the peptides are shown on the right. Both peptides bind anti-His antibody. However, whilst 4-8 binds to CEACAM1-Fc, 6-8 does not.

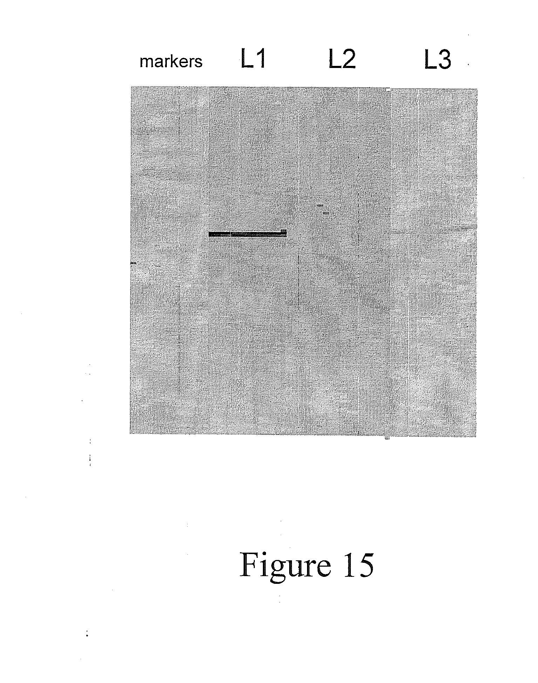

Figure 15 shows that D-8 polypeptide binds CEACAM1-Fc (lane 1) but not CD33-Fc used as a control (lane 2). The origin of this peptide was confirmed by reactivity with the anti-His tag antibody (lane 3).

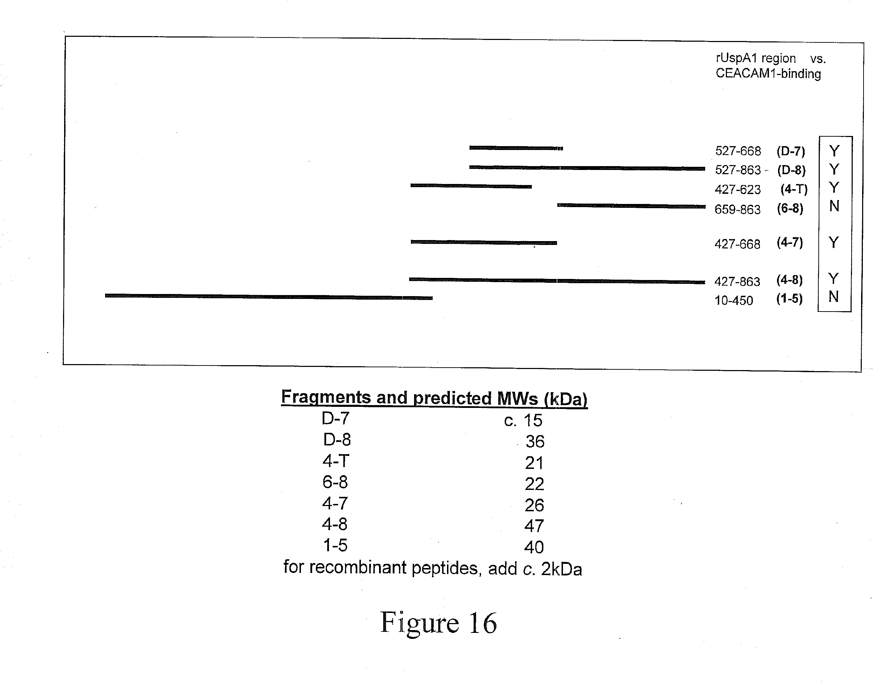

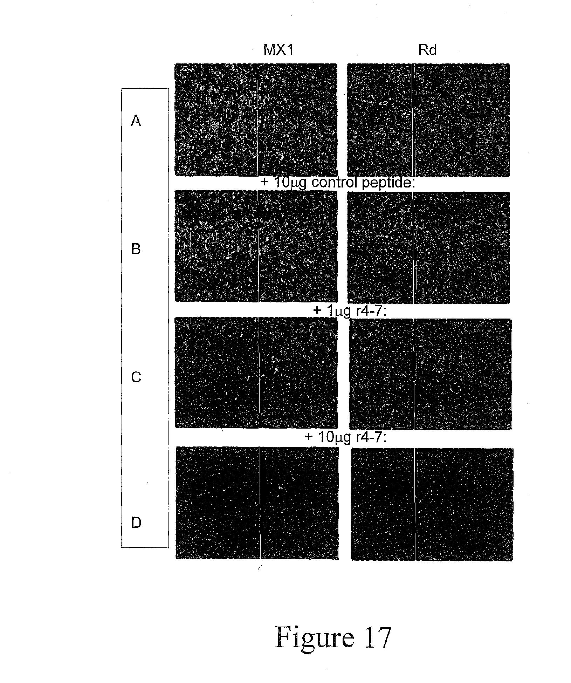

Figure 16 is a schematic diagram showing the relative sizes and positions of rUspA1 fragments. Recombinant 4-7 has been used for bacterial blocking - see Figure 17.
Figure 17 shows CHO-CEACAM1 transfectants were incubated in the absence of peptides (A) or with recombinant control peptide (a treponemal peptide, B) or UspA1 r4-7 peptide (sequence corresponding to the strain MX2, C and D) at the concentrations shown and bacteria added for a period of 2 hours. At the end of this incubation, unbound bacteria were washed off and bound bacteria detected with anti- *M*. *catarrhalis* polyclonal antiserum and TRITC conjugated secondary antibody. At 1 µg/ml significant inhibition of a heterologous *M. catarrhalis* strain (MX1) was obtained and at 10 µg per ml, *H. influenzae* binding was significantly inhibited by *M. catarrhalis* UspA1 recombinant peptide.

### Example 1. Moraxella catarrhalis strains bind to human CEACAM1-Fc via the UspA1 proteins

The strains of *Moraxella catarrhalis* (Mx) used in this study include clinical isolates (MX3 and MX4) as well as reference strain purchased from American Type Culture Collection (ATCC 25238, a clonal culture of this was designated MX2).

### Receptor overlay assays show that Mx bind to CEACAM1-Fc receptor constructs

To assess the interaction of *M. catarrhalis* strains with CEACAM1, bacteria (c. 4 - 8 x 10⁶) were applied to nitrocellulose, air dried and nonspecific binding sites blocked in 3 % BSA-PBST. Nitrocellulose strips were overlaid with either CEACAM1-Fc (1-2 µg ml⁻¹) alone or in the presence of the CEACAM1 N-domain antibody YTH71.3. CD33-Fc (1-2 µg ml⁻¹) was used as a negative control. Chimeric protein constructs were prepared as previously described¹¹. Chimeric receptor binding was detected by goat anti-human Fc antibodies conjugated to either horseradish peroxidase or alkaline phosphatase. Blots were developed with either diamino benzidene and hydrogen peroxide or nitroblue tetrazolium and 5-bromo-4-chloro-3-indoylphosphate substrates respectively. All Mx strains bound to CEACAM1-Fc but not to CD33-Fc (Fig.1). The receptor binding was inhibited in the presence the monoclonal antibody YTH71.3 suggesting that the strains bound to the N terminal domain of the receptor. Accordingly, all the strains bound equally well to the truncated N-Fc construct of CEACAM1, containing only the N domain (not shown).

### Identification of CEACAM1-Fc binding protein of M. catarrhalis

Whole cell lysates of Mx (c. 3 x 10⁷) were applied to individual lanes of a 10% bis-tris polyacrylamide gel (Invitrogen) either without previous heat treatment or after heating at 100°C for 10 minutes and subjected to electrophoresis for 45 min at 180 V. Proteins from the gels were transferred to nitrocellulose membranes using standard blotting conditions. Nitrocellulose membranes were overlaid with soluble chimeric constructs as described above. In each case, a single protein was observed that bound specifically to CEACAM1-Fc. The migration of the protein on gels was indicative of UspA1 protein of Mx, since when bacterial lysates were applied without heat denaturation, the CEACAM1 binding proteins migrated with an apparent mass of > 200 kDa whereas when the lysates were first heated, the CEACAM1-binding proteins migrated with a reduced mass of *circa* 92 kDa (Fig. 2)

### Antibodies raised against UspA1 but not those against a similar protein UspA2 bind to the CEACAM1-Fc binding proteins of Mx strains

Peptides were designed according to published sequences for the UspA proteins of *M. catarrhalis* strains. Namely, ETNNRQDQKIDQLGYALKEQGQ HFNNR (SEQ ID NO:1; UspA1-peptide) and KDEHDKLITANKTAIDANKAS (SEQ ID NO:6; UspA2-peptide). Peptides were coupled to KLH via an N-terminal cysteine residue that was incorporated and were used to immunise rabbits (200 µg peptide per rabbit) at 14 day intervals, initially with complete Freund's adjuvant and thereafter with incomplete Freund's adjuvant. Rabbits were bled at day 0 and at 14 day-intervals post immunisation. Polyclonal antibodies were purified using the appropriate peptide coupled to AminoLink Plus columns (Pierce). UspA-specific antibodies were used at a concentration of 1-10 µg ml⁻¹ for Western blot overlays and detected with goat anti-rabbit secondary antibody coupled to alkaline phosphatase. Blots were developed as described earlier. The migration of the CEACAM1-Fc binding proteins on SDS-PAGE of the three Mx strains was identical to that of the UspA1-antibody binding proteins but not UspA2-antibody binding proteins (Fig. 3)

### M. catarrhalis ligand co-precipitating with CEACAM1-Fc is identified as UspA1

Overnight cultures of bacteria were suspended in 100 mM octyl βD glucopyranoside in PBSB containing a protease inhibitor cocktail (pic; PMSF 1 mM, E-64 1 µM, pepstatin A 1 µM, bestatin 6 nM and EDTA 100 µM). Samples were mixed end over end overnight at 4°C. Meanwhile 100 µl protein A coupled to sepharose CL-4B (Sigma) was incubated with either, 20 µg CEACAM1-Fc or CD33-Fc (used as a control) overnight at 4°C and subsequently washed 3 times with PBSB to remove any unbound receptor. Insoluble bacterial material was removed by centrifugation at 15, 000 g for 30 min. Soluble extract was incubated with either receptor-Protein A sepharose complex for 2h at 4°C (at a ratio of 5 x10⁸ bacteria per µg of receptor construct). Following extensive washing with 50 mM octyl βD glucopyranoside and PBSB samples were analysed by SDS-PAGE electrophoresis and Western Blotting under denaturing conditions.

In co-precipitation experiments with CEACAM1-Fc, MX4 yielded a strongly staining protein of c. 97 kDa and MX3 yielded a relatively weakly staining protein of c. 92 kDa. The masses of co-precipitated proteins corresponded to those observed in the receptor overlay experiments (shown in Fig. 3). Neither protein co-precipitated with CD33-Fc. The co-precipitated proteins were further identified as UspA1 proteins since they bound to anti-UspA1 peptide antibody. In addition, following excision from the gel, the MX4 protein was subjected to MALDI-TOF mass spectrometry (see below).

### CEACAM1 ligand identification by MALDI-TOF mass spectrometry

(a) Western overlay samples. Whole cell lysates of MX2 and MX3 were subjected to SDS-PAGE in trench gels and the protein band corresponding to CEACAM1-Fc binding ligand was electroeluted from the gel. The sample was concentrated and reapplied in a single lane of a second gel, subjected to electrophoresis prior to in-gel trypsin digestion of the appropriate protein. The resulting peptides were analysed by Matrix-assisted Laser desorption/ionisation-time-of-flight (MALDI-TOF) mass spectrometry. An example of the resulting mass spectrum is shown for the CEACAM binding protein of MX2 (Fig 4a). The peptide masses obtained were entered into the ProFound protein identification site and the results were obtained as shown for this protein (Fig. 4b, c). In this case 10 peptide masses were matched to predicted masses for tryptic peptides of UspA1 of *M. catarrhalis* covering approximately 18 % of the protein (Fig. 4d). The estimated Z score of 2.34 is strongly suggestive of the protein being UspA1 (Z score >1.65 are above the 95^{th} percentile; http:/129.85.19.192/profound bin/webProFound.exe). In addition, another non-binding high molecular weight band of MX2 was identified as UspA2 following a similar analysis. Similarly for strain MX3, the CEACAM1 binding and non-binding proteins were identified as UspA1 and UspA2 respectively.
(b) Co-precipitated samples: For MX4, the CEACAM1-Fc co-precipitated protein (as described above) was also identified as UspA1 by MALDI-TOF MS with a Z score of 2.27 in an all taxa search. 12 peptides were matched covering 21 % of the protein.

This study therefore has identified that *Moraxella catarrhalis* targets human CEACAM1 via the high molecular weight protein UspA1 in the reference and clinical strains indicated.

### Enzymatic cleavage of UspA1 and recombinant peptide

### (a) Tryptic peptides of UspA1 bind to CEACAM1-Fc

MX2 bacterial suspensions (10¹⁰ ml⁻¹) were treated with Trypsin (Sigma) at 0.1-1 mg/ml concentrations and incubated for 1-4 hours at 37°C. Digested lysates were dissociated in SDS-PAGE buffer, boiled and subjected to electrophoresis. After transfer to nitrocellulose, the blots were overlaid with the receptor construct CEACAM1-Fc or the affinity purified anti-UspA1 peptide antibodies. A small fragment reacting with the receptor also bound to the anti-UspA1-specific antibodies (Fig. 5).

### (b) Localisation of CEACAM binding domain of UspA2 of M. catarrhalis strain MX2

MX2 bacterial suspensions were treated with Trypsin at 1mg/ml for 10 minutes at 37°C.

The trypsinised sample was run on an SDS-PAGE gel. After staining, the 50 kDa region was electroeluted overnight. Electroeluted protein was freeze dried and resuspended in buffer and applied to a second gel. Part of the gel was blotted onto nitrocellulose and peptide bands reacting with CEACAM were identified by Western blotting overlay using CEACAM1-Fc. (Fig.7).

Peptide bands corresponding to circa. 50 kDa and circa. 150 kDa peptides were subjected to N-terminal sequencing. The N-terminal sequences were ALESNVEEGL (SEQ ID NO:4) (*c*. 50 kDa peptide) and ALESNV (SEQ ID NO:7) (*c.* 150 kDa peptide). The 150 kDa protein is apparently a trimer of the 50 kDa protein as they have the same N-terminal sequence. The N-terminal sequence of this peptide of MX2 UspA1 is shown in Figure 8.

### (c) Recombinant peptide

The N-terminal recombinant MX2 peptide that was constructed consisting of amino acids 1 to 449 of the sequence shown in Figure 6 does not bind CEACAM. This further indicates that the *c.* 50 kDa tryptic peptide consisting of amino acids 463 to 863 of the sequence shown in Figure 8, having the N-terminal sequence ALESNVEEGL (SEQ ID NO:4) contains the CEACAM-binding domain.

### Example 2. Identification of Receptor Binding Domains on Multiple Virulence Determinants of Mucosal Pathogens

*N. meningitidis* and *H. influenzae* target human CEACAM molecules via ligands that bind to overlapping sites on CEACAM. The Mx ligand/s of the present invention also target the N domain. These observations point to an exciting possibility that similar features on ligands of several mucosal pathogens may be involved in receptor targeting.

Since Nm and Hi interactions with CEACAMs are affected by the structural features of the surface expressed variable domains, this suggests that they may contain similar crucial amino acids in a spatial configuration that are able to bind to the receptor and these may be conserved in otherwise variable proteins. Indeed our studies and those of others suggest that the two hyper-variable loops of Opa (HV1 and HV2) may be involved in CEACAM targeting ^{9,18} One powerful technique that can identify possible determinants of interactions between ligands and receptors is phage display, which can be used to identify mimotopes (random sequences to mimic the binding domains)¹⁹ as well as aptamers (sequences more closely related to the original structure that inhibit ligand binding) ²⁰.

The current invention also makes it highly feasible that the Mx ligand domain that binds CEACAMs will act as a mimic for other CEACAM-binding mucosal pathogens and the structural features of this ligand will help identify the salient features required in Nm and Hi ligands for CEACAM N-domain targeting. Antibodies to the Mx domain could have the potential of identifying other ligands that have the capacity to target the same, similar or closely positioned region of the receptor.

Identification of the minimal CEACAM1 binding domain of MX2 UspA1 is being undertaken using known methods of protein engineering and recombinant DNA technology. Recombinant peptides can be screened in vitro by receptor overlay assays described above to detect the domain of MX2 that binds to the receptor. His-Tagged peptides can be separated on a nickel column, His-Tag cleaved as required and used for immunising rabbits and mice to obtain antibodies for further investigations.

A peptide of suitable length for biological applications may be determined by examining immunological stimulatory properties as well other functions such as blocking of receptor binding.

### Example 3. Salient Features of the Receptor Required for Ligand Interactions

Since the N-domain of CEACAM is sufficient for the interactions of Opa proteins, the present inventors are using phage display technique also to investigate adhesive epitopes of the CEACAM N-domain. The knowledge of the ligand-binding region/s on the receptor, which has been studied by the present inventors by alanine scanning mutagenesis of the receptor⁹ has facilitated this study. In this case also, a ligand overlay assay is available for biopanning (affinity concentration) of chimeric phages that bear receptor sequences.

Receptor analogues have the potential to block multiple strains independently of the Opa type produced since our studies have already shown that despite their antigenic variation, distinct Opa proteins require common features on the receptor for primary adhesion⁹. It is also of interest to note that CEA antigens are shed from the gut mucosa and may block adhesion of *E. coli* strains also known to target CEACAMs. This has been proposed as a mechanism of innate immunity vs. enteric pathogens. ¹² Thus these receptor analogues act as therapeutic agents.

### Example 4. Production of Recombinant CEACAM-binding Moraxella catarrhalis UspA1 peptides

### Summary:

PCR amplification of several fragments along the length of UspA1 was carried out using the primers shown in Figure 9. Recombinant peptides were obtained as described below. In the first round, it was found that the recombinant peptide that bound to CEACAM1 was encoded by DNA amplified by primers P4 and P8 but not that encoded by region between P1 and P5. Further, recombinant P4 - P7 bound CEACAM1 but not P6 - P8. Additional primers were used within P4 and P7 region. Region D-7 retained CEACAM-binding. The sequence of fragment 4-7 is shown in Figure 10.

### General cloning, expression and purification strategy for recombinant UspA1 peptides

The UspA1 fragment 1-5 was produced using the pBAD system. The required PCR products were TA cloned into pBAD vector and TOP10 *E. coli* strain was used for amplification. The rest of the procedure was as shown in Fig. 11 with the exception that pBAD was induced using arabinose. The fragment 4-7 was produced using both the pBAD and pQE30 (Fig. 11) systems with similar CEACAM-binding results. The rest of the fragments were produced using the strategy in Fig. 11.

### Vector and the pQE30 expression system

The vector pQE30 (Fig. 12) was used in conjunction with *E. coli* strain M15. M15 contains the plasmid pREP4 encoding a repressor, which restricts transcription of DNA cloned into pQE30. The addition of IPTG to a concentration of 1mM prevents coding of this repressor and thus transcription of the cloned fragments in pQE30.

### The cloning strategy

PCR amplimers were first ligated into pCR2.1. This provided a stable host from which the amplimer could be recovered by restriction digest (*Bam*HI/*Pst*I), ensuring that each end of the gene fragment was cut. pQE30 was similarly digested and recovered by gel purification, which also ensured that both restriction sites had been cut. Digested pQE30 and UspA1 amplimer were ligated overnight at 16°C with T4 DNA ligase and transformed into CaCl₂ competent *E. coli* M15. Transformants were selected for on LB agar supplemented with ampicillin (100 µg/ml) and kanamycin (25 µg/ml). 4-8 colonies were picked and grown in LB broth with antibiotics. Bacteria from 3-4ml of culture were collected by centrifugation and subjected to the alkaline-lysis miniprep method. Purified vector was digested as above to check for *uspA1* insert. Bacteria containing pQE30 with a correct sized insert were grown in 50 ml. cultures and induced with IPTG (see below). Western blots were then used to screen for recombinant protein production. In addition, vectors were sequenced to determine that the insert was the correct region of DNA and to check for any sequence errors.

### Expression and purification

M15 containing pQE30/*uspA1* constructs were grown in LB (supplemented with 100 µg/ml Ampicillin and 25 µg/ml Kanamycin) broth with shaking to OD600 = 0.5 before the addition of IPTG to a concentration of 1 mM. Cultures were incubated for a further 3-4 hours and bacteria recovered by centrifugation. Bacterial pellets were solubilised in buffer B (8M urea, 50mM Tris, 10% ethanol, 2% Tween, 5mM imidazole, pH 7) for 1-3 hrs and membrane material removed by centrifugation at 20,000 g for 20 min. Supernatants were incubated with nickel resin for 1-2 hrs on a rotary mixer, and passed through a polypropylene column. The retained resin was washed with 5-10 ml buffer B and bound protein eluted with 0.5 ml elutions of buffer B supplemented with 100 mM imidazole. Eluted proteins were checked by SDS-PAGE before dialysis to remove urea and other salts.

### Recombinant fragments

### A: Fragments 1-5 and 4-8

These fragments produced by pBAD system showed that 1-5 did not bind CEACAM1 but 4-8 did.

### B: Fragments 4-8, 4-8T, 4-7 and 6-8 produced using pQE30 system.

Fragment 4-8 was the first rUspA1 fragment produced and was found to bind CEACAM1 with a high affinity in blot-overlay assays. In addition to the full-length 4-8 rUspA1 peptide, a smaller, truncated, protein was observed. This peptide (designated 4-T) also bound CEACAM1 and appeared to be expressed at lower levels than 4-8 (Fig. 13). Sequence analysis of pQE30/4-T found that a mismatch (CAA to TAA) led to a termination codon at residue Q624 (see Fig. 10).

Peptides 4-7 and 6-8 were produced in order to rule out the possibility that a second CEACAM binding site occurred in 6-8. As predicted from the 4-T peptide, 4-7 demonstrated CEACAM binding (Fig. 13) but 6-8 did not (Fig. 14).

### C: Fragments D-8 and D-7 produced by pQE30 system

Both rUspA1 fragments D-8 (Fig. 15) and D-7 (not shown) were found to bind CEACAM1.

### Biological activity of recombinant peptide 4-7

*M. catarrhalis* strain MX1 and *H. influenzae* strain Rd bind to Chinese Hamster Ovary (CHO) cells transfected with CEACAM1. Their binding can be blocked with *M. catarrhalis* UspA1 recombinant peptide 4-7 but not a control peptide (Fig. 17).

### References:

1. Cartwright, K. et al. 1995. In Meningococcal Disease. John Wiley & Sons.
2. van Alphen, L. & van Ham, S. M.. 1994. Rev Med Microbiol 5:245.
3. Foxwell, A. R. et al. 1998. Microbiol Mol Biol Rev 62:294.
4. van Alphen, L. et al. 1995. Am J Respir Crit Care Med 151:2094.
5. Karalus, R. et al. 2000. Microbes & Infection 2:5
6. Kraft, M. 2000. Clin Chest Med 21:301.
7. Virji, M. et al. 1996. Mol Microbiol 22:929.
8. Virji, M. et al. 1996. Mol Microbiol 22:941.
9. Virji, M. et al. 1999. Mol Microbiol 34:538.
10. Virji, M. et al. 2000. Mol Microbiol 36:784.
11. Hill, D. et al. 2001 Mol Microbiol 39: 850.
12. Hammarström, S. 1999 Cancer Biol 9:67.
13. Stephens, D. S. 1989. Clin Microbiol Rev 2:S104.
14. Virji, M. et al. 1991. Mol Microbiol 5:1831.
15. Achtman, M. 1995 Trends Microbiol 3:186.
16. Merz, A. J. & So, M. 2000 Annu Rev Cell Dev Biol 16:423.
17. Woods, J. P. & Cannon, J. G. 1990 Infect Immun 58:569.
18. Wang, L-F & Yu, M. 1996 In Methods Mol Biol 66:269 (Morris, G. E. ed). Humana Press.
19. Colman-Lerner. 2000. Trends Guide p. 56 (Wilson, E. ed. ).
20. Beauchemin, N., et al., 1999 Exp Cell Res 252: 243-249.
21. Boulton I.C. & Gray-Owen S.D. 2002 Nat Immunol 3(3):229-36.
22. Chen T. et al., 2001 J Leukoc Biol 70(2):335-40.
23. Lafontaine, E.R., et al., 2000 J Bacteriol 182: 1364-1373.
24. Virji, M. 2001 Trends in Microbiology, 9: 258-259.

## Claims

1. A ligand comprising a CEACAM receptor binding domain, said ligand consisting of an amino acid sequence selected from the group consisting of residues 463 to 863, 527 to 668, 527 to 863, 427 to 623, 427 to 668, and 427 to 863 of the sequence shown in Figure 6, or a fragment, homologue, functional equivalent, derivative, degenerate or hydroxylation, sulphonation or glycosylation product or other secondary processing product thereof.

2. A medicament comprising the ligand according to claim 1 and one or more pharmaceutically acceptable adjuvants, vehicles, excipients, binders, carriers, or preservatives.

3. A ligand according to claim 1 for use in the treatment or prophylaxis of infection.

4. A ligand according to claim 1 for use in the treatment or prevention of any disease where CEACAM receptors are implicated.

5. A ligand according to claim 4 for the use in treatment or prevention of infection, respiratory disease, neoplastic diseases and associated conditions of neoplastic diseases, and angiogenesis.

6. A ligand according to any one of claims 3 to 5, where the infection is of, or has occurred via, a mucosal membrane.

7. A ligand according to claim 6, wherein the infection is caused by *Neisseria meningitidis, Haemophilus influenzae* or *Moraxella catarrhalis.*

8. A ligand according to claim 1 for use in the prophylaxis or treatment of otitis media.

9. A screening assay for the identification of novel blocking reagents for use as therapeutic agents comprising screening potential therapeutic agents for their ability to mimic, or for their homology to, the ligand according to claim 1.

10. A vaccine comprising the ligand according to claim 1 and one or more pharmaceutically acceptable adjuvants, vehicles, excipients, binders, carriers, or preservatives.

11. Use of a CEACAM receptor-binding ligand in the manufacture of a medicament for the treatment or prophylaxis of a disease in which CEACAM receptors are involved in cellular targeting of the pathogen which causes the disease, wherein the ligand comprises an amino acid sequence selected from the group consisting of residues 463 to 863, 527 to 668, 527 to 863, 427 to 623, 427 to 668, and 427 to 863 of the sequence shown in Figure 6, or a fragment, homologue, functional equivalent, derivative, degenerate or hydroxylation, sulphonation or glycosylation product or other secondary processing product thereof.

12. Use according to claim 11 wherein the disease is selected from the group consisting of infection, respiratory disease, neoplastic disease and associated conditions of neoplastic disease, and angiogenesis.

13. Use according to claim 11 or 12, wherein the pathogen infects, or enters via, a mucosal membrane.

14. Use according to any of claims 11 to 13, wherein the pathogen which causes the disease is selected from the group consisting of *Neisseria meningitidis, Haemophilus influenzae* and *Moraxella catarrhalis.*

15. Use according to claim 14, wherein the disease is otitis media.


SEQUENCE LISTING

<110> University of Bristol
      Virji, Mumtaz

<120> Therapeutic peptides

<130> APM/8950-WO

<140> PCT/GB2003/004273
<141> 2003-10-01

<150> GB 0222764.3
<151> 2002-10-02

<160> 7

<170> PatentIn version 3.1

<210> 1
<211> 27
<212> PRT
<213> Moraxella catarrhalis

<400> 1

Glu Thr Asn Asn His Gln Asp Gln Lys Ile Asp Gln Leu Gly Tyr Ala
1               5                   10                  15

Leu Lys Glu Gln Gly Gln His Phe Asn Asn Arg
            20                  25

<210> 2
<211> 863
<212> PRT
<213> Moraxella catarrhalis

<400> 2

Met Asn Lys Ile Tyr Lys Val Lys Lys Asn Ala Ala Gly His Leu Val
1               5                   10                  15

Ala Cys Ser Glu Phe Ala Lys Gly His Thr Lys Lys Ala Val Leu Gly
            20                  25                  30

Ser Leu Leu Ile Val Gly Ile Leu Gly Met Ala Thr Thr Ala Ser Ala
        35                  40                  45

Gln Lys Val Gly Lys Ala Thr Asn Lys Ile Ser Gly Gly Asp Asn Asn
    50                  55                  60

Thr Ala Asn Gly Thr Tyr Leu Thr Ile Gly Gly Gly Asp Tyr Asn Lys
65                  70                  75                  80

Thr Lys Gly Arg Tyr Ser Thr Ile Gly Gly Gly Leu Phe Asn Glu Ala
                85                  90                  95

Thr Asn Glu Tyr Ser Thr Ile Gly Ser Gly Gly Tyr Asn Lys Ala Lys
            100                 105                 110

Gly Arg Tyr Ser Thr Ile Gly Gly Gly Gly Tyr Asn Glu Ala Thr Asn
        115                 120                 125

Gln Tyr Ser Thr Ile Gly Gly Gly Asp Asn Asn Thr Ala Lys Gly Arg
    130                 135                 140

Tyr Ser Thr Ile Gly Gly Gly Gly Tyr Asn Glu Ala Thr Ile Glu Asn
145                 150                 155                 160

Ser Thr Val Gly Gly Gly Gly Tyr Asn Gln Ala Lys Gly Arg Asn Ser
                165                 170                 175

Thr Val Ala Gly Gly Tyr Asn Asn Glu Ala Thr Gly Thr Asp Ser Thr
            180                 185                 190

Ile Ala Gly Gly Arg Lys Asn Gln Ala Thr Gly Lys Gly Ser Phe Ala
        195                 200                 205

Ala Gly Ile Asp Asn Lys Ala Asn Ala Asp Asn Ala Val Ala Leu Gly
    210                 215                 220

Asn Lys Asn Thr Ile Glu Gly Glu Asn Ser Val Ala Ile Gly Ser Asn
225                 230                 235                 240

Asn Thr Val Lys Lys Gly Gln Gln Asn Val Phe Ile Leu Gly Ser Asn
                245                 250                 255

Thr Asp Thr Thr Asn Ala Gln Asn Gly Ser Val Leu Leu Gly His Asn
            260                 265                 270

Thr Ala Gly Lys Ala Ala Thr Ile Val Asn Ser Ala Glu Val Gly Gly
        275                 280                 285

Leu Ser Leu Thr Gly Phe Ala Gly Ala Ser Lys Thr Gly Asn Gly Thr
    290                 295                 300

Val Ser Val Gly Lys Lys Gly Lys Glu Arg Gln Ile Val His Val Gly
305                 310                 315                 320

Ala Gly Glu Ile Ser Asp Thr Ser Thr Asp Ala Val Asn Gly Ser Gln
                325                 330                 335

Leu His Ala Leu Ala Thr Val Val Ala Gln Asn Lys Ala Asp Ile Lys
            340                 345                 350

Asp Leu Asp Asp Glu Val Gly Leu Leu Gly Glu Glu Ile Asn Ser Leu
        355                 360                 365

Glu Gly Glu Ile Phe Asn Asn Gln Asp Ala Ile Ala Lys Asn Gln Ala
    370                 375                 380

Asp Ile Lys Thr Leu Glu Ser Asn Val Glu Glu Gly Leu Leu Asp Leu
385                 390                 395                 400

Ser Gly Arg Leu Ile Asp Gln Lys Ala Asp Ile Asp Asn Asn Ile Asn
                405                 410                 415

Asn Ile Tyr Glu Leu Ala Gln Gln Gln Asp Gln His Ser Ser Asp Ile
            420                 425                 430

Lys Thr Leu Lys Asn Asn Val Glu Glu Gly Leu Leu Asp Leu Ser Gly
        435                 440                 445

Arg Leu Ile Asp Gln Lys Ala Asp Leu Thr Lys Asp Ile Lys Ala Leu
    450                 455                 460

Glu Ser Asn Val Glu Glu Gly Leu Leu Asp Leu Ser Gly Arg Leu Ile
465                 470                 475                 480

Asp Gln Lys Ala Asp Ile Ala Lys Asn Gln Ala Asp Ile Ala Gln Asn
                485                 490                 495

Gln Thr Asp Ile Gln Asp Leu Ala Ala Tyr Asn Glu Leu Gln Asp Ala
            500                 505                 510

Tyr Ala Lys Gln Gln Thr Glu Ala Ile Asp Ala Leu Asn Lys Ala Ser
        515                 520                 525

Ser Ala Asn Thr Asp Arg Ile Ala Thr Ala Glu Leu Gly Ile Ala Glu
    530                 535                 540

Asn Lys Lys Asp Ala Gln Ile Ala Lys Ala Gln Ala Asn Glu Asn Lys
545                 550                 555                 560

Asp Gly Ile Ala Lys Asn Gln Ala Asp Ile Gln Leu His Asp Lys Lys
                565                 570                 575

Ile Thr Asn Leu Gly Ile Leu His Ser Met Val Ala Arg Ala Val Gly
            580                 585                 590

Asn Asn Thr Gln Gly Val Ala Thr Asn Lys Ala Asp Ile Ala Lys Asn
        595                 600                 605

Gln Ala Asp Ile Ala Asn Asn Ile Lys Asn Ile Tyr Glu Leu Ala Gln
    610                 615                 620

Gln Gln Asp Gln His Ser Ser Asp Ile Lys Thr Leu Ala Lys Val Ser
625                 630                 635                 640

Ala Ala Asn Thr Asp Arg Ile Ala Lys Asn Lys Ala Glu Ala Asp Ala
                645                 650                 655

Ser Phe Glu Thr Leu Thr Lys Asn Gln Asn Thr Leu Ile Glu Gln Gly
            660                 665                 670

Glu Ala Leu Val Glu Gln Asn Lys Ala Ile Asn Gln Glu Leu Glu Gly
        675                 680                 685

Phe Ala Ala His Ala Asp Val Gln Asp Lys Gln Ile Leu Gln Asn Gln
    690                 695                 700

Ala Asp Ile Thr Thr Asn Lys Thr Ala Ile Glu Gln Asn Ile Asn Arg
705                 710                 715                 720

Thr Val Ala Asn Gly Phe Glu Ile Glu Lys Asn Lys Ala Gly Ile Ala
                725                 730                 735

Thr Asn Lys Gln Glu Leu Ile Leu Gln Asn Asp Arg Leu Asn Arg Ile
            740                 745                 750

Asn Glu Thr Asn Asn His Gln Asp Gln Lys Ile Asp Gln Leu Gly Tyr
        755                 760                 765

Ala Leu Lys Glu Gln Gly Gln His Phe Asn Asn Arg Ile Ser Ala Val
    770                 775                 780

Glu Arg Gln Thr Ala Gly Gly Ile Ala Asn Ala Ile Ala Ile Ala Thr
785                 790                 795                 800

Leu Pro Ser Pro Ser Arg Ala Gly Glu His His Val Leu Phe Gly Ser
                805                 810                 815

Gly Tyr His Asn Gly Gln Ala Ala Val Ser Leu Gly Ala Ala Gly Leu
            820                 825                 830

Ser Asp Thr Gly Lys Ser Thr Tyr Lys Ile Gly Leu Ser Trp Ser Asp
        835                 840                 845

Ala Gly Gly Leu Ser Gly Gly Val Gly Gly Ser Tyr Arg Trp Lys
    850                 855                 860

<210>  3
<211>  401
<212>  PRT
<213>  Moraxella catarrhalis

<400>  3

Ala Leu Glu Ser Asn Val Glu Glu Gly Leu Leu Asp Leu Ser Gly Arg
1               5                   10                  15

Leu Ile Asp Gln Lys Ala Asp Ile Ala Lys Asn Gln Ala Asp Ile Ala
            20                  25                  30

Gln Asn Gln Thr Asp Ile Gln Asp Leu Ala Ala Tyr Asn Glu Leu Gln
        35                  40                  45

Asp Ala Tyr Ala Lys Gln Gln Thr Glu Ala Ile Asp Ala Leu Asn Lys
    50                  55                  60

Ala Ser Ser Ala Asn Thr Asp Arg Ile Ala Thr Ala Glu Leu Gly Ile
65                  70                  75                  80

Ala Glu Asn Lys Lys Asp Ala Gln Ile Ala Lys Ala Gln Ala Asn Glu
                85                  90                  95

Asn Lys Asp Gly Ile Ala Lys Asn Gln Ala Asp Ile Gln Leu His Asp
            100                 105                 110

Lys Lys Ile Thr Asn Leu Gly Ile Leu His Ser Met Val Ala Arg Ala
        115                 120                 125

Val Gly Asn Asn Thr Gln Gly Val Ala Thr Asn Lys Ala Asp Ile Ala
    130                 135                 140

Lys Asn Gln Ala Asp Ile Ala Asn Asn Ile Lys Asn Ile Tyr Glu Leu
145                 150                 155                 160

Ala Gln Gln Gln Asp Gln His Ser Ser Asp Ile Lys Thr Leu Ala Lys
                165                 170                 175

Val Ser Ala Ala Asn Thr Asp Arg Ile Ala Lys Asn Lys Ala Glu Ala
            180                 185                 190

Asp Ala Ser Phe Glu Thr Leu Thr Lys Asn Gln Asn Thr Leu Ile Glu
        195                 200                 205

Gln Gly Glu Ala Leu Val Glu Gln Asn Lys Ala Ile Asn Gln Glu Leu
    210                 215                 220

Glu Gly Phe Ala Ala His Ala Asp Val Gln Asp Lys Gln Ile Leu Gln
225                 230                 235                 240

Asn Gln Ala Asp Ile Thr Thr Asn Lys Thr Ala Ile Glu Gln Asn Ile
                245                 250                 255

Asn Arg Thr Val Ala Asn Gly Phe Glu Ile Glu Lys Asn Lys Ala Gly
            260                 265                 270

Ile Ala Thr Asn Lys Gln Glu Leu Ile Leu Gln Asn Asp Arg Leu Asn
        275                 280                 285

Arg Ile Asn Glu Thr Asn Asn His Gln Asp Gln Lys Ile Asp Gln Leu
    290                 295                 300

Gly Tyr Ala Leu Lys Glu Gln Gly Gln His Phe Asn Asn Arg Ile Ser
305                 310                 315                 320

Ala Val Glu Arg Gln Thr Ala Gly Gly Ile Ala Asn Ala Ile Ala Ile
                325                 330                 335

Ala Thr Leu Pro Ser Pro Ser Arg Ala Gly Glu His His Val Leu Phe
            340                 345                 350

Gly Ser Gly Tyr His Asn Gly Gln Ala Ala Val Ser Leu Gly Ala Ala
        355                 360                 365

Gly Leu Ser Asp Thr Gly Lys Ser Thr Tyr Lys Ile Gly Leu Ser Trp
    370                 375                 380

Ser Asp Ala Gly Gly Leu Ser Gly Gly Val Gly Gly Ser Tyr Arg Trp
385                 390                 395                 400

Lys

<210>  4
<211>  10
<212>  PRT
<213>  Moraxella catarrhalis

<400>  4

Ala Leu Glu Ser Asn Val Glu Glu Gly Leu
1               5                   10

<210>  5
<211>  242
<212>  PRT
<213>  Moraxella catarrhalis

<400>  5

Gln His Ser Ser Asp Ile Lys Thr Leu Lys Asn Asn Val Glu Glu Gly
1               5                   10                  15

Leu Leu Asp Leu Ser Gly Arg Leu Ile Asp Gln Lys Ala Asp Leu Thr
            20                  25                  30

Lys Asp Ile Lys Ala Leu Glu Ser Asn Val Glu Glu Gly Leu Leu Asp
        35                  40                  45

Leu Ser Gly Arg Leu Ile Asp Gln Lys Ala Asp Ile Ala Lys Asn Gln
    50                  55                  60

Ala Asp Ile Ala Gln Asn Gln Thr Asp Ile Gln Asp Leu Ala Ala Tyr
65                  70                  75                  80

Asn Glu Leu Gln Asp Ala Tyr Ala Lys Gln Gln Thr Glu Ala Ile Asp
                85                  90                  95

Ala Leu Asn Lys Ala Ser Ser Ala Asn Thr Asp Arg Ile Ala Thr Ala
            100                 105                 110

Glu Leu Gly Ile Ala Glu Asn Lys Lys Asp Ala Gln Ile Ala Lys Ala
        115                 120                 125

Gln Ala Asn Glu Asn Lys Asp Gly Ile Ala Lys Asn Gln Ala Asp Ile
    130                 135                 140

Gln Leu His Asp Lys Lys Ile Thr Asn Leu Gly Ile Leu His Ser Met
145                 150                 155                 160

Val Ala Arg Ala Val Gly Asn Asn Thr Gln Gly Val Ala Thr Asn Lys
                165                 170                 175

Ala Asp Ile Ala Lys Asn Gln Ala Asp Ile Ala Asn Asn Ile Lys Asn
            180                 185                 190

Ile Tyr Glu Leu Ala Gln Gln Gln Asp Gln His Ser Ser Asp Ile Lys
        195                 200                 205

Thr Leu Ala Lys Val Ser Ala Ala Asn Thr Asp Arg Ile Ala Lys Asn
    210                 215                 220

Lys Ala Glu Ala Asp Ala Ser Phe Glu Thr Leu Thr Lys Asn Gln Asn
225                 230                 235                 240

Thr Leu

<210> 6
<211> 21
<212> PRT
<213> Moraxella catarrhalis

<400> 6

Lys Asp Glu His Asp Lys Leu Ile Thr Ala Asn Lys Thr Ala Ile Asp
1               5                   10                  15

Ala Asn Lys Ala Ser
            20

<210> 7
<211> 6
<212> PRT
<213> Moraxella catarrhalis

<400> 7

Ala Leu Glu Ser Asn Val
1               5